# EUROPÄISCHE PATENTSCHRIFT

(11) **EP 1 720 466 B1**
(45) Veröffentlichungstag und Bekanntmachung des Hinweises auf die Patenterteilung: **23.04.2008**
(21) Anmeldenummer: 05716681.1
(22) Anmeldetag: 14.02.2005
(51) Int. Cl.: A61B 17/22

(54) **ANLAGE ZUR NICHTINVASIVEN MEDIZINISCHEN BEHANDLUNG**
NON-INVASIVE MEDICAL TREATMENT INSTALLATION
EQUIPEMENT POUR UN TRAITEMENT MEDICAL NON INVASIF

(30) Priorität: 01.03.2004 DE 102004010004
(43) Veröffentlichungstag der Anmeldung: 15.11.2006
(73) Patentinhaber: Siemens Aktiengesellschaft, 80333 München (DE)
(72) Erfinder: KALTSCHMIDT, Rainer, 90542 Eckental/Brand (DE); WILL, Ulrich, 91358 Kunreuth (DE)
(86) Internationale Anmeldenummer: PCT/EP2005/050629
(87) Internationale Veröffentlichungsnummer: WO 2005/082260

(56) Entgegenhaltungen:
- DE-A1- 19 808 402
- DE-C1- 10 106 832
- US-A- 5 044 354
- US-A- 5 199 420
- US-A1- 2003 078 523

## Beschreibung

Die Erfindung betrifft eine Anlage zur nichtinvasiven medizinischen Behandlung, bei der ein Therapiegerät, etwa ein Stoßwellenkopf im Falle einer Lithotripsiebehandlung auf einer Kreisbahn um einen Patiententisch bzw. um einen auf diesem gelagerten Patienten herum geführt wird. Eine zu behandelnde Körperregion des Patienten wird dabei im Isozentrum der genannten Kreisbahn angeordnet. Der Fokus des Therapiegerätes, im Falle eines Stoßwellenkopfes also der Fokus der von ihm ausgehenden Ultraschallwellen, befindet sich dabei im Isozentrum bzw. in der zu behandelnden Körperregion. Zur Führung des Therapiegerätes wird im allgemeinen ein kreisförmiger Bogen, ein sogenannter C-Bogen verwendet. Bei einem fest am Grundgestell fixierten C-Bogen muss dieser eine Bogenlänge aufweisen, die mindestens so groß ist wie der gewünschte Verfahrweg des Therapiegerätes. Die Bogenlänge des C-Bogens kann verkürzt werden, wenn dieser am Grundgestell orbital verfahrbar gelagert ist. Ein verfahrbar an einem C-Bogen geführtes Therapiegerät hat den Vorteil, das es auf unterschiedlichen Körperseiten eines Patienten positionierbar ist, ohne das der Patient auf dem Patiententisch umgelagert werden muss. Eine Anlage der in Rede stehenden Art ist in der Regel so aufgebaut, dass das Grundgestell und weitere Anlagenteile auf einer Seite des Patiententisches angeordnet sind, wobei die andere Seite des Patiententisches im wesentlichen frei bleiben soll, um einen behinderungsfreien Zugang zum Patienten, etwa zu Anästhesiezwecken zu ermöglichen. Soll nun ein Therapiegerät auf dieser Seite des Patiententisches in Position gebracht werden, so stört weniger das Therapiegerät selbst, weil dieses relativ nahe am Patienten positioniert wird, als vielmehr der C-Bogen. Wird beispielsweise zur Lithotripsiebehandlung ein Stoßwellenkopf in der 0° Position, d.h. in der Obertischposition bei vertikaler Ausrichtung seiner Stoßwellenachse positioniert, so ragt der C-Bogen mindestens bis zu dieser Winkelstellung in den Raum oberhalb des Patienten hinein. Ein behandelnder Arzt ist dadurch im Bereich seines Kopfes in seiner Bewegungsfreiheit stark eingeschränkt.

Dokument US-A-5044354 offenbart eine Anlage gemäss dem Oberbegriff des ersten Anspruchs.

Davon ausgehend ist es die Aufgabe der Erfindung, eine Anlage zur nichtinvasiven medizinischen Behandlung vorzuschlagen, mit der dieser Nachteil umgangen ist.

Diese Aufgabe wird nach Anspruch 1 gelöst. Danach ist am Therapie-C-Bogen ein Fixierende und ein Freiende aufweisender Tragarm angeordnet, wobei der Tragarm mit seinem Fixierende zwischen zwei von den Bogenenden vorgegebenen Endstellung orbital verfahrbar am Therapie-C-Bogen gelagert ist und an seinem Freiende das Therapiegerät trägt. Der Tragarm ist weiterhin um eine Drehachse drehbar am Therapie-C-Bogen gelagert, wobei er in beiden Endstellungen derart ausrichtbar ist, dass er sich über das jeweilige Bogenende hinaus erstreckt. Die drehbare Lagerung des Tragarmes am Therapie-C-Bogen gewährleistet, dass auch in der jeweils anderen Endstellung ein solcher Überstand über das Bogenende hinaus auf einfache Weise herstellbar ist.

Vorzugsweise ist die Drehachse des Tragarmes so ausgerichtet, dass sie den Fokus des Therapiegerätes schneidet. Dadurch ist gewährleistet, dass bei einer Drehung etwa um 180° um die Drehachse der Therapiefokus seine Position nicht verändert wird. Üblicherweise liegt dieser im Isozentrum eines C-Bogens. Die Lage des Therapiefokus wird somit weder durch eine Orbitalbewegung des Tragarmes noch durch eine Drehung um die Drehachse verändert.

Bei einer weiteren bevorzugten Ausgestaltung ist das Therapiegerät so angeordnet, dass sich sein Fokus in einer Ebene befindet, die parallel und beabstandet zur Orbitalebene des Therapie-C-Bogens verläuft. Durch diese Ausgestaltung ist es möglich, den Wirkort des Therapiegerätes von der Orbitalebene des Therapie-C-Bogens zu entfernen und dadurch im Bereich des Therapie-C-Bogens noch mehr Bewegungsfreiheit für eine den Patienten betreuende Person zu schaffen. Besonders vorteilhaft ist diese Ausgestaltung jedoch dann, wenn zu einer behandlungsbegleitenden Bildgebung ein Röntgen-C-Bogen koaxial, planparallel und mit axialem Versatz zum Therapie-C-Bogen angeordnet ist, wobei der Fokus des Therapiegerätes mit dem Isozentrum des Röntgen-C-Bogens zusammenfällt. Neben der schon erwähnten erhöhten Bewegungsfreiheit für medizinisches Personal ergibt sich dabei der Vorteil, dass der Röntgen-C-Bogen praktisch behinderungsfrei orbital verfahrbar ist. Bei einer Anordnung von Röntgen- und Therapie-C-Bögen, bei der die Orbitalebene der beiden Bögen zusammenfallen, ist die orbitale Verfahrbarkeit des Röntgen-C-Bogens erheblich eingeschränkt, z.B. weil Röntgenquelle oder Röntgenempfänger in die Bewegungsbahn des Therapie-C-Bogens hineinragen.

Die Erfindung wird nun anhand eines in den beigefügten Zeichnungen dargestellten Ausführungsbeispiels näher erläutert. Es zeigen:
- Fig. 1: in perspektivischer Darstellung ein Grundgestell einer Anlage zur therapeutischen Behandlung, an der ein ein Therapiegerät tragender Therapie-C-Bogen orbital bzw. um ein Isozentrum verfahrbar gelagert ist,
- Fig. 2: einen Ausschnitt aus Fig. 1 in perspektivischer Darstellung,
- Fig. 3: einen Ausschnitt aus einer Anlage in perspektivischer Darstellung, bei der dem Therapie-C-Bogen ein Röntgen-C-Bogen beigeordnet ist,
- Fig. 4: eine Abbildung entsprechend Fig. 1, bei der sich das Therapiegerät in einer anderen Position befindet,
- Fig. 5: eine schematische perspektivische Darstellung, welche die Position des Therapiegerätes von Fig. 4 in einer anwendungsbezogenen Situation zeigt,
- Fig. 6: eine schematische Darstellung eines Therapie-C-Bogens und eines beigeordneten Röntgen-C-Bogens.

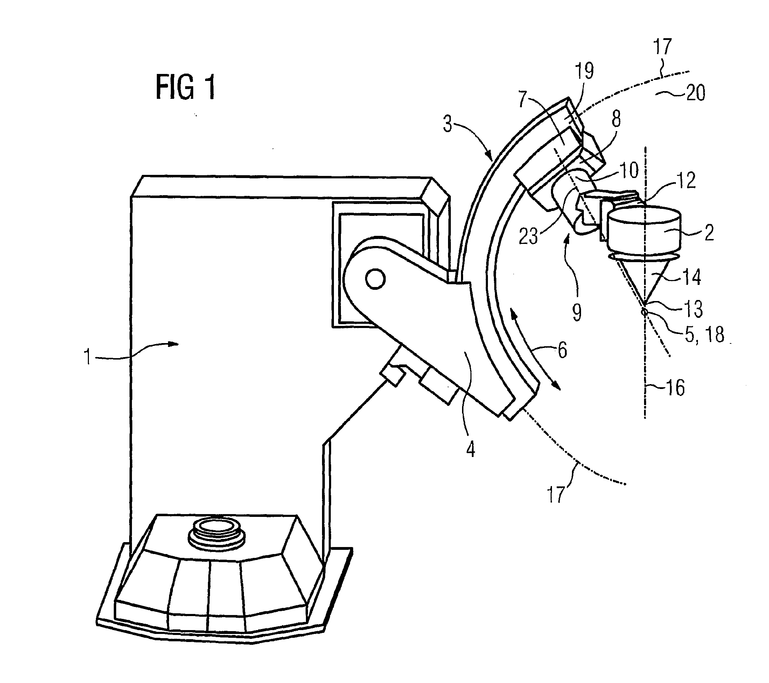

Eine in den Abbildungen dargestellte Anlage umfasst ein Grundgestell 1, an dem ein erster C-Bogen fixiert ist, der ein Therapiegerät, beispielsweise den Stosswellenkopf 2 einer Lithotripsieanlage trägt. Der erste C-Bogen, im folgenden mit Therapie-C-Bogen 3 bezeichnet, ist ein Kreisringsegment, welches an einem Ausleger 4 des Grundgestells 1 um seinen Mittelpunkt bzw. um sein Isozentrum 5 orbital verfahrbar ist, was in Fig. 1 durch den Doppelpfeil 6 angedeutet ist. Am Therapie-C-Bogen 3 ist ein Schlitten 7 orbital, also entsprechend Doppelpfeil 6, verfahrbar gelagert. An einer zum Isozentrum 5 weisenden Seite 8 des Schlittens ist ein Tragarm 9 mit seinem Fixierende 10 befestigt. Das Freiende 12 des Tragarms 9 trägt den Stosswellenkopf 2. Aufgrund der orbitalen Beweglichkeit des Therapie-C-Bogens 3 und des Schlittens 7 kann der Stosswellenkopf 2 in verschiedenen Winkelposition zum Isozentrum 5 bzw. zu einem Patiententisch 15 positioniert werden. Der Radialabstand des Stosswellenkopfes 2 zum Isozentrum 5 ist so gewählt, dass der Fokus 13 eines vom Stosswellenkopf 2 ausgesandten Schallwellenkegels 14 auf einer sich durch das Isozentrum 5 hindurch erstreckenden Zentralachse 18 liegt. Der Stosswellenkopf 2 kann dabei beispielsweise so angeordnet sein, dass seine Stosswellenachse 16 in der vom Therapie-C-Bogen 3 aufgespannten Orbitalebene 17 verläuft.

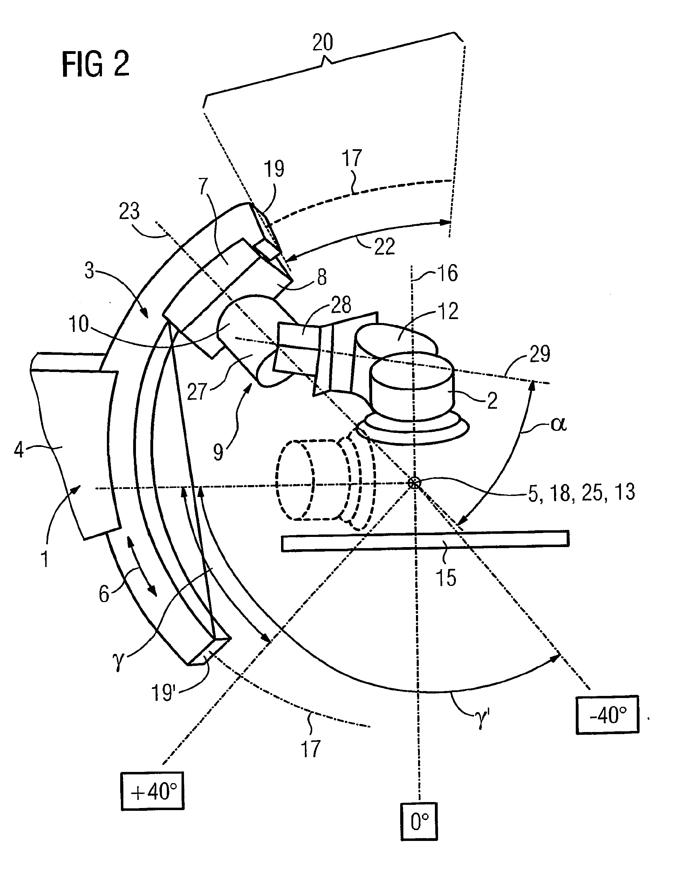

Wie aus Fig. 1 und insbesondere Fig. 2 erkennbar ist, ist der Tragarm 9 so ausgestaltet bzw. so ausgerichtet, dass er sich - in Richtung der senkrecht auf der Orbitalebene 17 stehenden und sich durch das Isozentrum 5 hindurcherstreckenden Zentralachse 18 gesehen - in seiner oberen Endstellung (Fig. 2) über das obere Bogenende 19 in Bogenumfangsrichtung hinaus erstreckt. Dadurch entsteht oberhalb des Stoßwellenkopfes 2 ein behinderungsfrei zugänglicher Raum 20 im Kopfbereich einer einen Patienten während der Behandlung betreuenden Person. Wäre der Tragarm - ebenfalls in der Projektion der Fig. 2 gesehen - etwa in Richtung der Stoßwellenachse 16, also radial ausgerichtet, müsste der Therapie-C-Bogen 3 etwa um das Bogensegment 22 länger sein bzw. um eine entsprechende Strecke weiter orbital verfahren werden, wobei er im Raum 20 die Bewegungsfreiheit einer betreuenden Person einschränken würde.

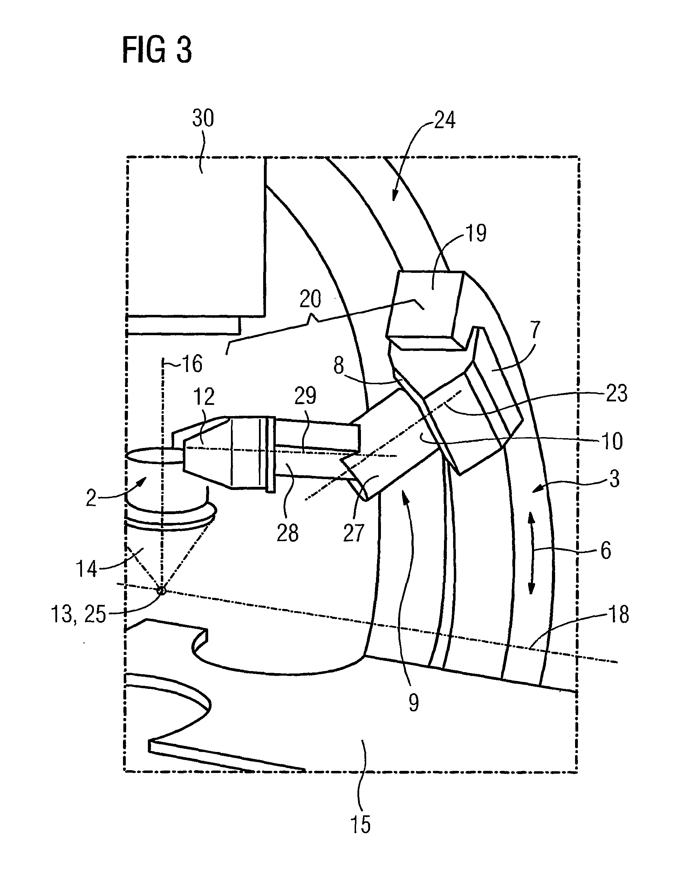

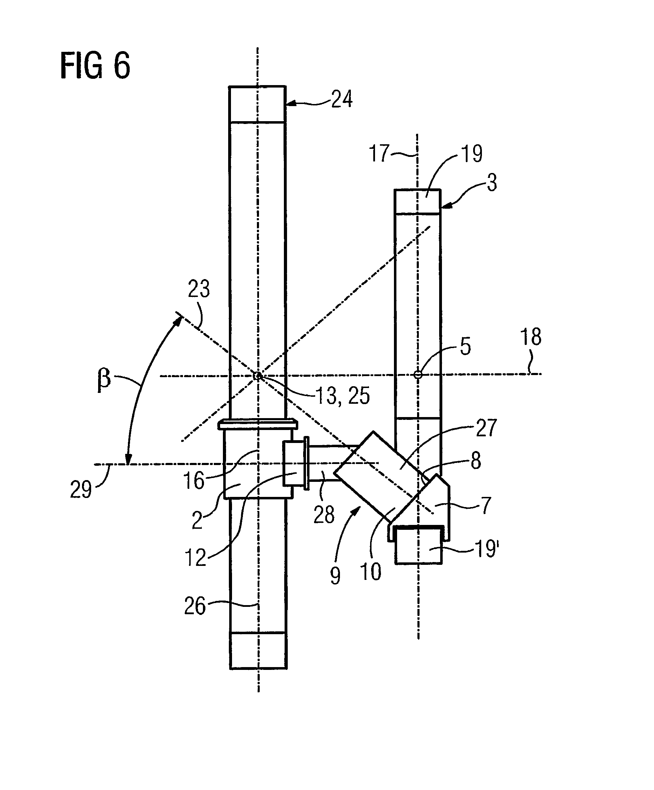

Damit auch in der unteren Endstellung sowohl des Therapie-C-Bogens 3 als auch des Schlittens 7 der Tragarm 9 das untere Bogenende 19'in Bogenumfangsrichtung überragt, ist der Tragarm 9 um eine Drehachse 23 drehbar am Schlitten 7 gelagert. Es wäre nun denkbar, dass nicht nur eine einzige Drehachse vorhanden ist, sondern das auch der Stoßwellenkopf 2 gegenüber dem Tragarm 9 einen Freiheitsgrad aufweist. Zweck einer solchen Beweglichkeit wäre es, den Stoßwellenkopf 2 nach Durchlauf eines orbitalen Verfahrweges wieder in eine Position zu bringen, in der sein Fokus 13 wieder auf der Zentralachse 18 zu liegen kommt, beispielsweise mit dem Isozentrum 5 des Therapie-C-Bogens 3 zusammenfällt. Mehrere Bewegungsmöglichkeiten bzw. Gelenkstellen bilden aber Fehlerquellen hinsichtlich einer exakten Ausrichtung des Stoßwellenkopfes 2 in Folge von Toleranzen, die sich bei beweglich miteinander verbundenen Teilen nie ganz ausschließen lassen. Bei den beschriebenen Ausführungsbeispielen ist daher der Stoßwellenkopf 2 starr mit dem ebenfalls starr ausgebildeten Tragarm 9 verbunden. Die Drehung des Tragarmes 9 erfolgt um eine einzige Achse, nämlich die Drehachse 23. Bei einer Drehung um 180°C um diese Achse befindet sich der Tragarm, ebenso wie der Stoßwellenkopf, in einer zur vorherigen Position spiegelbildlichen Ausrichtung, wobei die Drehachse 23 die Spiegelachse bildet. Zur Röntgen-unterstützten Beobachtung etwa einer Lithotripsiebehandlung kann dem Therapie-C-Bogen ein diesen koaxial umfassender eine Röntgenquelle (nicht dargestellt) und einen Röntgenempfänger 30 tragender Röntgen-C-Bogen ohne oder mit axialem Versatz beigestellt werden. Im ersten Falle fallen die Orbitalebenen und die Isozentren der beiden C-Bögen zusammen. Dementsprechend verläuft die Drehachse 23 des Tragarms 9 in der gemeinsamen Orbitalebene der C-Bögen und erstreckt sich durch deren gemeinsames Isozentrum. Der Stoßwellenkopf 2 kann dabei so ausgerichtet werden, dass seine Stoßwellenachse 16 in der gemeinsamen Orbitalebene verläuft. Bei einer Drehung um die Drehachse 23 um 180° beim Übergang von einer Endstellung in die andere nimmt der Stoßwellenkopf 2 wieder eine Position ein, bei der seine Stoßwellenachse 16 in der Orbitalebene 17 des Therapie-C-Bogens 3 verläuft. Die Beobachtung mit dem Röntgensystem kann dann in jeder Winkelposition "inline", d.h. in Richtung der Stoßwellenachse 16 erfolgen. Im zweiten, in den Zeichnungen dargestellten Fall ist der Röntgen-C-Bogen 24 mit Axialabstand zum Therapie-C-Bogen 3 angeordnet. Sein Isozentrum 25 liegt, ebenso wie das Isozentrum 5 des Therapie-C-Bogens 3, auf der Zentralachse 18. Der Tragarm 9 erstreckt sich, wie insbesondere Fig. 3 und Fig. 6 zu entnehmen ist, seitlich aus der Orbitalebene 17 heraus. Der am Freiende 12 des Tagarms 9 fixierte Stoßwellenkopf 2 ist dann im Bereich der Orbitalebene 26 des Röntgen-C-Bogens 24 angeordnet, wobei sich sein Fokus 13 in dessen Isozentrum 25 befindet. Der Stoßwellenkopf 2 kann so ausgerichtet werden, dass in einer Winkelstellung je Seite seine Stoßwellenachse 16 in der Orbitalebene 26 des Röntgen-C-Bogens 24 verläuft. Bei einer Drehung um die Drehachse 23 verändert sich aber diese Ausrichtung, d.h. die Stoßwellenachse 16 wird aus der Orbitalebene 26 heraus gekippt, wobei, wie erfindungsgemäß beabsichtigt, das gemeinsame Isozentrum erhalten bleibt.

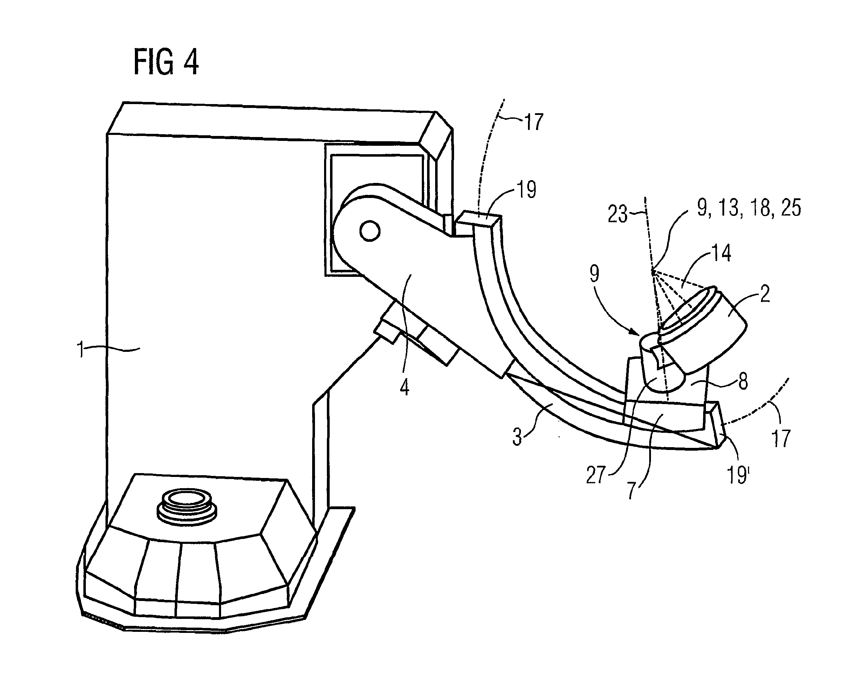

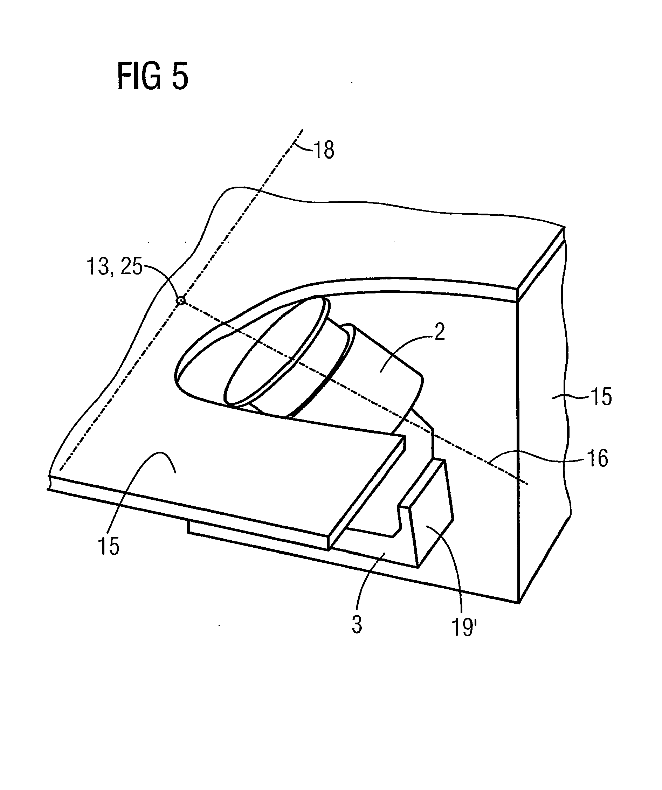

Der Tragarm 9 weist einen ersten, das Fixierende 10 beinhaltenden Längsabschnitt 27 und einen zweiten das Freiende 12 beeinhaltenden Längsabschnitt 28 auf. Der Längsabschnitt 27 ist drehbar am Schlitten 7 gelagert. Die Drehachse 23, die identisch mit der Mittellängsachse 29 des Längsabschnitts 27 ist, durchstößt mit ihrem einen Ende die Orbitalebene 17 des Therapie-C-Bogens 3 und schneidet das Isozentrum 25 des Röntgen-C-Bogens 24. Bei einer Orbitalverschiebung des Schlittens 7 auf dem Therapie-C-Bogen 3 überstreicht die Drehachse 23 die Mantelebene eines Kegelsegments, dessen Basisfläche von der Orbitalebene 17 des Therapie-C-Bogens und dessen Spitze vom Isozentrum 25 des Röntgen-C-Bogens 24 gebildet wird. Die Seite 8 des Schlittens 7, aus welcher der Längsschnitt 26 hervorsteht, verläuft rechtwinklig zur Drehachse 23. Der zweite Längsabschnitt 28 ist schräg am ersten Längsabschnitt 27 fixiert. Dabei bildet seine Mittellängsachse 29 in der Projektion auf die Orbitalebene 17 mit der Drehachse 23 einen spitzen Winkel α (Fig.2) und in der Projektion auf eine von der Drehachse 23 und der Zentralachse 18 aufgespannte Ebene einen spitzen Winkel β (Fig.6). Wenn ausgehend von der Obertisch-Position der Fig. 1-3 der Stoßwellenkopf 2 in eine Untertisch-Position (Fig. 4-6) verfahren werden soll, etwa zur Behandlung einer linken oder rechten Niere, sind zwei symmetrische Operationen erforderlich, nämlich zum einen eine Drehung um bis zu 180° um die Drehachse 23 und eine Orbitalverschiebung des Schlittens 7. Obwohl die beiden Bewegungsabläufe natürlich simultan ablaufen können, werden sie zur besseren Verständlichkeit nacheinander beschrieben. Beginnt man ausgehend von der Situation in Fig.2 zunächst mit einer Drehung um die Drehachse 23 um 180°, so nimmt danach der Stoßwellenkopf 2 etwa die mit gestrichelten Linien dargestellte Position ein. Wie Fig. 2 entnehmbar ist, entspricht dies einer Drehung um die Zentralachse 18. Der Fokus 13 verharrt bei der Drehung im Isozentrum 25. Die Stoßwellenachse 16 überstreicht dabei die Mantelfläche eines Kegelsegments, dessen Spitze das Isozentrum 25 ist. Soll der Stoßwellenkopf 2 etwa in der +40°-Position ausgerichtet werden, so ist, ausgehend von der in gestrichelten Linien gezeigten Position, eine Orbitalverschiebung um etwa 50° (Winkel γ) und bei einer Ausrichtung in der -40°-Position um etwa 130° Winkel γ') erforderlich. Bei einem etwa radial ausgerichteten, also sich in Richtung der Stoßwellenachse 16 erstreckenden Tragarm wäre hingegen ein orbitaler Verfahrweg des Schlittens 7 - ebenfalls ausgehend von der Obertisch-Position - von etwa 240° erforderlich. Dazu wäre ein Therapie-C-Bogen 3 mit einer Bogenlänge von mehr als 120° nötig. Bei einer erfindungsgemäßen Ausgestaltung des Tragarms 9 dagegen kann der Therapie-C-Bogen 3 etwa um ein dem Bogenstück 22 entsprechendes Stück verkürzt werden.

## Patentansprüche

1. Anlage zur nichtinvasiven medizinischen Behandlung mit einem an einem Grundgestell (1) angeordneten, ein Isozentrum (5) aufweisenden Therapie-C-Bogen (3) und einem einen Fokus (13) aufweisenden Therapiegerät, mit folgender weiteren Ausgestaltung:
- am Therapie-C-Bogen (3) ist ein ein Fixierende (10) und ein Freiende (12) aufweisender Tragarm (9) angeordnet,
- der Tragarm (9) ist mit seinem Fixierende (10) zwischen zwei von den Bogenenden (19, 19') begrenzten Endstellungen am Therapie-Bogen (3) orbital verfahrbar gelagert und trägt an seinem Freiende (12) das Therapiegerät, **dadurch gekennzeichnet, dass**
- der Tragarm (9) um eine Drehachse (23) drehbar am Therapie-C-Bogen (3) gelagert ist, wobei er in beiden Endstellungen derart ausrichtbar ist, dass er sich über das jeweilige Bogenende (19, 19') hinaus erstreckt.

2. Anlage nach Anspruch 1, **dadurch gekennzeichnet, dass** die Drehachse (23) des Tragarms (9) den Fokus (13) des Therapiegerätes schneidet.

3. Anlage nach Anspruch 1 oder 2, **dadurch gekennzeichnet, dass** das Therapiegerät so angeordnet ist, dass sich sein Fokus (13) in einer Ebene befindet, die parallel und beabstandet zur Orbitalebene (17) des Therapie-C-Bogens (3) verläuft.

4. Anlage nach Anspruch 3, **gekennzeichnet durch** einen Röntgen-C-Bogen (24), der koaxial, planparallel und mit axialem Versatz zum Therapie-C-Bogen (3) angeordnet ist, wobei der Fokus (13) des Therapiegerätes mit dem Isozentrum (25) des Röntgen-C-Bogens (24) zusammenfällt.

## Claims

1. Non-invasive medical treatment installation with a therapy C-arm (3) having an isocentre (5) arranged on a base unit (1) and a therapy device having a focus (13), with the following further embodiment:
- Arranged on the therapy C-arm (3) is a carrier arm (9) having a fixing end (10) and a free end (12),
- the carrier arm (9) is able to be moved with its fixing end (10) orbitally between two end positions delimited by the C-arm ends (19, 19') on the therapy C-arm (3) and supports at its free end (12) the therapy device,
**characterised in that**
- the carrier arm (9) is supported on the therapy C-arm (3) to allow rotation around an axis of rotation (23), with the carrier arm being able to be aligned in the two end positions such that it extends beyond the respective C-arm end (19, 19').

2. Installation according to claim 1, **characterised in that** the axis or rotation (23) of the carrier arm (9) intersects the focus (13) of the therapy device.

3. Installation according to claim 1 or 2, **characterised in that** the therapy device is arranged so that its focus (13) is in a plane which runs in parallel at a distance from the orbital plane (17) of the therapy C-arm (3).

4. Installation according to claim 3, **characterised by** an x-ray C-arm (24) which is arranged coaxially, in a parallel plane and with an axial offset to the therapy C-arm (3), with the focus (13) of the therapy device coinciding with the isocentre (25) of the x-ray C-arm (24).

## Revendications

1. Installation de traitement médical non invasif, comportant un arc de traitement (3) placé sur un support de base (1) et muni d'un isocentre (5) et un appareil de traitement muni d'un foyer (13), la configuration de l'installation étant en outre la suivante :
- un bras porteur (9) muni d'une extrémité de fixation (10) et d'une extrémité libre (12) est placé sur l'arc de traitement (3),
- par son extrémité de fixation (10) le bras porteur (9) est monté sur l'arc de traitement (3) de manière à se déplacer en sens orbital entre deux positions terminales délimitées par les extrémités d'arc (19, 19'),
**caractérisé en ce que** le bras porteur (9) est monté sur l'arc de traitement (3) de façon à pouvoir tourner autour d'un axe de rotation (23), en pouvant être orienté dans les deux positions terminales de telle manière qu'il dépasse de l'extrémité d'arc (19, 19') correspondante.

2. Installation selon la revendication 1, **caractérisée en ce que** l'axe de rotation (23) du bras porteur (9) coupe le foyer (13) de l'appareil de traitement.

3. Installation selon la revendication 1 ou 2, **caractérisée en ce que** l'appareil de traitement est disposé de telle manière que son foyer (13) se trouve dans un plan parallèle au plan orbital (17) de l'appareil de traitement (3) et à distance de lui.

4. Installation selon la revendication 3, **caractérisée par** un arc de radiographie (24) qui par rapport à l'arc de traitement (3) est disposé coaxialement, dont les faces lui sont parallèles et qui est décalé axialement, le foyer (13) de l'appareil de traitement coïncidant avec l'isocentre (25) de l'arc de radiographie (24) aux rayons X.
